**Europäisches Patentamt**

(19) **European Patent Office**

**Office européen des brevets**

(11) Veröffentlichungsnummer: **0 001 741**
**B1**

(12) **EUROPÄISCHE PATENTSCHRIFT**

(45) Veröffentlichungstag der Patentschrift:
22.04.81

(51) Int. Cl.³: **G 01 N 33/48, A 61 B 5/14**

(21) Anmeldenummer: **78100771.1**

(22) Anmeldetag: **28.08.78**

(54) Mittel zur Erleichterung der Auszählung von Thrombozyten in Blutproben.

(30) Priorität: **07.10.77 DE 2745151**

(43) Veröffentlichungstag der Anmeldung:
**16.05.79 Patentblatt 79/10**

(45) Bekanntmachung des Hinweises auf die Patenterteilung:
**22.04.81 Patentblatt 81/16**

(84) Benannte Vertragsstaaten:
**BE CH FR GB NL SE**

(56) Entgegenhaltungen:
**US-A-3 045 494**
**US-A-3 574 137**
**US-A-3 632 735**

(73) Patentinhaber: **Walter Sarstedt**
**Kunststoff-Spritzgusswerk,**
**D-5223 Nümbrecht/Rommelsdorf (DE)**

(72) Erfinder: **Sarstedt, Walter,**
**D-5223 Nümbrecht/Rommelsdorf (DE)**
Erfinder: **Rodjak, Djuro, Konrad-Adenauer-Strasse 17,**
**D-7208 Spaichingen (DE)**

(74) Vertreter: **Dahlke, Werner, Dipl.-Ing. et al, Frankenforster Strasse 137, D-5060 Bergisch Gladbach 3 (DE)**

ACTORUM AG.

Mittel zur Erleichterung der Auszählung von Thrombozyten in Blutproben

Die Erfindung betrifft ein Mittel zur Erleichterung der Auszählung von Thrombozyten in Gegenwart von Erythrozyten in Blutproben.

Bei der direkten Auszählung von Thrombozyten in Blutproben unter dem Mikroskop stören die gleichzeitig anwesenden Erythrozyten deshalb besonders stark, weil sie kräftig rot gefärbt sind und zum Teil die Thrombozyten überdecken. Die Auszählung von Thrombozyten nach dieser direkten Methode war deshalb bisher stets mit einer recht grossen Unsicherheit behaftet bzw. kaum exakt möglich.

Die Erfindung hat die Aufgabe, die Auszählung der Thrombozyten in Gegenwart der störenden Erythrozyten zu verbessern.

Zur Lösung dieser Aufgabe wird ein Mittel vorgeschlagen, das in einer wässerigen Lösung von Quecksilber-II-Chlorid in einer Konzentration zwischen 1,2 und 4,0 g/l besteht, deren pH-Wert zwischen 1,5 und 4,8 eingestellt ist. Eine solche wässerige Lösung dient dann zur Verdünnung des zu untersuchenden Blutes, wobei zweckmässig 2 ml dieser Lösung mit 20 µl des zu untersuchenden Blutes vermischt werden. Das Mittel bewirkt, dass die Erythrozyten entfärbt werden, während die Thrombozyten im mikroskopischen Bild leuchtender werden, so dass sie relativ leicht auszuzählen sind.

Das Mischungsverhältnis zwischen der Menge der Lösung und der Blutmenge ist für ein Blut mit etwa normalen Thrombozytengehalt optimal, da durch die so erzielte Verdünnung eine sichere Auszählung mit einer minimalen Fehlerquote möglich ist. In Sonderfällen kann von diesem Mischungsverhältnis allerdings auch abgegangen werden, wie weiter unten noch dargelegt wird.

Vorzugsweise wird vorgeschlagen, dass der pH-Wert zwischen 2,0 und 4,5 liegt. Innerhalb dieses engeren pH-Bereiches ist eine besonders gute Zählung der Thrombozyten möglich.

Vorzugsweise wird ferner vorgeschlagen, dass die Konzentration der Lösung an Quecksilber-II-Chlorid zwischen 1,5 und 3,5 g/l liegt. In diesem Bereich ist die Leuchtkraft der Thrombozyten sehr gut und dadurch die Auszählung besonders erleichtert.

Der optimale Wert innerhalb der vorgenannten Bereiche liegt bei einem pH-Wert von 2,5 und bei einer Konzentration an Quecksilber-II-Chlorid von 2,75 g/l.

Diese Werte sind auch aus einem anderen Grunde optimal: falls bei pathologisch vermindertem Thrombozytengehalt die Auszählung im Mikroskop bei dem angegebenen Mischungsverhältnis unsicher wird, weil sich nur einige wenige Thrombozyten im Bild befinden, kann man auf eine höhere Blutkonzentration, von 50 µl oder gegebenenfalls sogar 100 µl Blut auf die angegebene Menge von 2 ml der Lösung übergehen. Dann steigt die Zahl der Thrombozyten im mikroskopischen Bildfeld und die Auszählung wird sicherer. Gleichzeitig wird aber infolge des höheren pH-Wertes des Blutes (oberhalb 7,0) auch der pH-Wert der Mischung stärker nach oben (in Richtung auf den Neutralpunkt) verschoben, als es durch die kleine Blutmenge von 20 µl der Fall war. Der als optimal angegebene pH-Wert von 2,5 liegt aber so niedrig, dass auch bei Zugabe von 100 µl Blut zu 2 ml Lösung der pH-Wert der Mischung nicht über 4,5 steigt. Im allgemeinen bleibt der pH-Wert der Mischung sogar unter 4,0.

Für den Fall, dass eine grössere Blutmenge zu der eingangs vorgeschlagenen Lösung in ihrem weiten Konzentrationsbereich zugegeben werden soll, wird ganz allgemein vorgeschlagen, dass der pH-Wert der Lösung so niedrig eingestellt wird, dass der pH-Wert der Mischung nach Blutzugabe unter 4,5, vorzugsweise unter 4,0 bleibt.

Der pH-Wert wird vorzugsweise durch Zugabe von Salzsäure oder Salpetersäure oder auch von Quecksilber-II-Nitrat eingestellt.

Beispiel

In 1000 ml destilliertes Wasser wurden 2,75 g Quecksilber-II-Chlorid gegeben und bis zur Lösung gerührt. Sodann wurde der pH-Wert durch Zugabe von Salzsäure auf 2,5 eingestellt.

20 µl Blut wurden zu 2,0 ml der vorgenannten Lösung gegeben und vorsichtig bis zur Entfärbung der Erythrozyten (Hämolyse) gemischt. Aus dieser Mischung wurden die Thrombozyten mit Hilfe einer Zählkammermethode mikroskopisch ausgezählt.

Die Untersuchungen erfolgten bei Raumtemperatur. Ein Einfluss der Temperatur auf die Wirksamkeit des Mittels wurde nicht beobachtet. Bei diesem Versuch wurde Venenblut verwendet, das mit di-K-EDTA ungerinnbar gemacht worden war.

**Patentansprüche**

1. Mittel zur Erleichterung der Auszählung von Thrombozyten in Gegenwart von Erythrozyten in Blutproben, gekennzeichnet durch eine wässerige Lösung von Quecksilber-II-Chlorid in einer Konzentration zwischen 1,2 und 4,0 g/l, deren pH-Wert zwischen 1,5 und 4,8 eingestellt ist.

2. Mittel nach Anspruch 1, dadurch gekennzeichnet, dass der pH-Wert im Bereich zwischen 2,0 und 4,5 liegt.

3. Mittel nach Anspruch 1 und 2, dadurch gekennzeichnet, dass die Konzentration an Quecksilber-II-Chlorid in der Lösung zwischen 1,5 und 3,5 g/l liegt.

4. Mittel nach Anspruch 1 bis 3, dadurch gekennzeichnet, dass der pH-Wert 2,5 beträgt und die Konzentration an Quecksilber-II-Chlorid 2,75 g/l ist.

5. Mittel nach Anspruch 1 und 2, bestimmt für die Zugabe einer grösseren Blutmenge, dadurch gekennzeichnet, dass der pH-Wert der Lösung innerhalb des genannten Bereiches so niedrig eingestellt ist, dass er nach Blutzugabe nicht über 4,5, vorzugsweise nicht über 4,0, steigt.

6. Mittel nach Anspruch 1 bis 5, dadurch gekennzeichnet, dass der pH-Wert durch Zugabe von Salzsäure eingestellt ist.

7. Mittel nach Anspruch 1 bis 5, dadurch gekennzeichnet, dass der pH-Wert durch Zugabe von Salpetersäure eingestellt ist.

8. Mittel nach Anspruch 1 bis 5, dadurch gekennzeichnet, dass der pH-Wert durch Zugabe von Quecksilber-II-Nitrat eingestellt ist.

## Claims

1. Agent for facilitating the counting of thrombocytes in the presence of erythrocytes in blood samples, characterised by an aqueous solution which contains mercury-II chloride in a concentration of between 1.2 and 4.0 g/l, and whose pH value is set to between 1.5 and 4.8.

2. Agent according to Claim 1, characterised in that the pH value lies in the range between 2.0 and 4.5.

3. Agent according to Claim 1 and 2, characterised in that the concentration of mercury-II chloride in the solution lies between 1.5 and 3.5 g/l.

4. Agent according to Claim 1 to 3, characterised in that the pH value is 2.5 and the concentration of mercury-II chloride is 2.75 g/l.

5. Agent according to Claim 1 and 2, intended for the addition of a sizable amount of blood, characterised in that the pH value of the solution is set to a sufficiently low value, within the stated range, that after addition of the blood it does not rise above 4.5, and preferably not above 4.0.

6. Agent according to Claim 1 to 5, characterised in that the pH value is set by addition of hydrochloric acid.

7. Agent according to Claim 1 to 5, characterised in that the pH value is set by addition of nitric acid.

8. Agent according to Claim 1 to 5, characterised in that the pH value is set by addition of mercury-II nitrate.

## Revendications

1. Milieu destiné à faciliter le comptage de thrombocytes en présence d'érythrocytes dans des échantillons de sang, caractérisé par une solution aqueuse de chlorure mercurique à une concentration comprise entre 1,2 et 4,0 g/l, solution dont le pH est réglé à une valeur se situant entre 1,5 et 4,8.

2. Milieu selon la revendication 1, caractérisé en ce que le pH se situe dans la gamme comprise entre 2,0 et 4,5.

3. Milieu selon la revendication 1 ou 2, caractérisé en ce que la concentration de la solution en chlorure mercurique se situe entre 1,5 et 3,5 g/l.

4. Milieu selon l'une quelconque des revendications 1 à 3, caractérisé en ce que le pH a une valeur de 2,5 et la concentration en chlorure mercurique est de 2,75 g/l.

5. Milieu selon la revendication 1 ou 2, destiné à l'addition d'une plus grande quantité de sang, caractérisé en ce que le pH de la solution est réglé, dans la gamme mentionnée, à une valeur suffisamment basse pour qu'après l'addition de sang, il ne s'élève pas au-dessus de 4,5 et, de préférence, pas au-dessus de 4,0.

6. Milieu selon l'une quelconque des revendications 1 à 5, caractérisé en ce que le pH est réglé par addition d'acide chlorhydrique.

7. Milieu selon l'une quelconque des revendications 1 à 5, caractérisé en ce que le pH est réglé par addition d'acide nitrique.

8. Milieu selon l'une quelconque des revendications 1 à 5, caractérisé en ce que le pH est réglé par addition de nitrate mercurique.